# EUROPÄISCHE PATENTANMELDUNG

(11) **EP 4 574 130 A1**
(43) Veröffentlichungstag der Anmeldung: **25.06.2025**
(21) Anmeldenummer: 24222355.0
(22) Anmeldetag: 20.12.2024
(51) Int. Cl.: A61K 8/24, A61K 8/44, A23L 33/16, A61K 9/00, A61K 31/198, A61K 45/00, A61P 17/14, A61Q 5/00, A61Q 7/00

(54) **ZUSAMMENSETZUNG ZUR TOPISCHEN BEHANDLUNG VON EPITHELGEWEBE**

(30) Priorität: 22.12.2023 DE 102023005341
(71) Anmelder: Dr. Kurt Wolff GmbH & Co. KG, 33611 Bielefeld (DE)
(72) Erfinder: SCHULZE ZUR WIESCHE, Erik, 33611 Bielefeld (DE); POLY, Wolfgang, 33611 Bielefeld (DE)
(74) Vertreter: Weickmann & Weickmann PartmbB

(57) **Zusammenfassung**

Die vorliegende Erfindung betrifft eine Zusammensetzung zur Behandlung des menschlichen Epithelgewebes umfassend wenigstens eine organische Säure mit mindestens zwei funktionellen Gruppen, wobei wenigstens eine funktionelle Gruppe eine Carbonsäure ist, ein in Wasser lösliches anorganisches mehrwertiges Salz, und sowie deren Verwendung.

## Beschreibung

Die vorliegende Erfindung betrifft eine Zusammensetzung zur Behandlung des menschlichen Epithelgewebes umfassend wenigstens eine organische Säure mit mindestens zwei funktionellen Gruppen, wobei wenigstens eine funktionelle Gruppe eine Carbonsäure ist, und ein in Wasser lösliches anorganisches mehrwertiges Salz, und sowie deren Verwendung.

Der Begriff "Epithelgewebe" bezeichnet Schichten sogenannter Epithelzellen, die eng miteinander verbunden sind. Es handelt sich um eine der vier Grundgewebearten - die anderen drei sind Muskel-, Nerven- und das Bindegewebe. Das Epithelgewebe besitzt einige charakteristische Eigenschaften: Epithelien enthalten etwa keine Blutgefäße. Die Zellen des Epithelgewebes sind durch die Basalmembran vom Bindegewebe getrennt und werden durch diese mit Nährstoffen versorgt.

Das Epithelgewebe bedeckt innere und äußere Körperoberflächen - so besteht beispielsweise die Haut als größtes Organ des Menschen aus Epithelgewebe.

Unter ihren vielen Funktionen (beispielsweise zur Wärmeregulation und als Sinnesorgan) ist die Barriere- und Schutzfunktion, die das Austrocknen (und damit letztlich des gesamten Organismus) verhindert, die wohl Wichtigste Aufgabe der Haut. Gleichzeitig wirkt die Haut als Schutz gegen das Eindringen und die Aufnahme von externen Stoffen. Insbesondere sind hier schädliche UV-Strahlen zu nennen, mikrobiologische Angriffe und Penetration von hautfremden Schadstoffen. Bewirkt wird diese Barrierefunktion durch die Epidermis (Oberhaut), welche als äußerste Schicht die eigentliche Schutzhülle gegenüber der Umwelt bildet.

Besondere Beachtung gilt der äußeren Hornschicht (Stratum Comeum), einer widerstandsfähigen Zellschicht, deren einzelne Hornzellen über Corneodesmosomen unter Beteiligung von Cadherinen, Calciumionen (Ca²⁺)-abhängigen transmembranen Glykoproteinen aus der Gruppe der Adhäsionsproteine, miteinander verbunden sind. Zwischen und auf den Hornzellen befindet sich eine Lipidschicht, welche insbesondere einen physikalischen und chemischen Schutz bietet und den Zusammenhalt der Hornschicht unterstützt.

Der natürliche pH-Wert der Hautoberfläche liegt in der Regel zwischen pH 4-7. Mit etwa einem Zehntel der Gesamtdicke der Haut ist die Epidermis gleichzeitig die dünnste Schicht der Haut. Die Melanozyten (Pigmentzellen) in der Basalzellschicht liefern die Pigmente, die der Haut ihre Färbung verleihen. Diese umfasst neben der Epidermis noch das darunter gelegene mesodermale Bindegewebe des Coriums (Lederhaut, Unterhaut). Beide Schichten sind durch ein System papillen- oder leistenformiger Coriumsvorwölbungen und tief zwischen diese hineinragender Epidermiszapfen innig miteinander verzahnt. Vom reich durchbluteten Corium her erfolgt auch die Nährstoffversorgung der Epidermis. In den Coriumpapillen liegen die Sinnesrezeptoren der Hautsinne für Tastempfindung, Schmerzempfindung und Temperaturempfindung.

Die oberflächlichen Zelllagen der Epidermis unterliegen einem ständigen Verschleiß. Zusätzlich kann die Oberhaut sehr empfindlich mit Reizungen, Rötungen, Juckreiz, Schuppungen und Rissbildungen auf äußere Einflüsse reagieren. Zudem ist sie permanent der Gefahr von Entzündungen, und Verletzungen (Wunden) ausgesetzt. Um daher den Schutz optimal zu gewährleisten, erneuert sich die Epidermis deshalb innerhalb von ca. 30 Tagen vollständig. Diese Epidermiserneuerung erfolgt aus einer basalen, zeitlebens teilungsaktiven Keimschicht durch eine kontinuierliche Ersetzung.

Kosmetische und dermatologische Zubereitungen für die Pflege der Haut sind bekannt. So basieren derartige Zusammensetzungen auf der Grundlage von O/W - oder W/O-Emulsionen, die beispielsweise auf einer Mischung aus langkettigen Fettsäuren, Mono- und/oder Diglyceriden von Fettsäuren, ethoxylierten Fettsäureestem, unpolaren Lipiden, Fettalkoholen, lipophilen Konsistenzgebern, hydrierten Polyisobutenen und polaren Lipiden basieren.

Insbesondere um die Hautbarriere der Epidermis zu verbessern, werden seit Jahrzenten viele kosmetische Zusammensetzungen angeboten, durch welche üblicherweise von außen Fettstoffe der Haut zugeführt werden. Solche Zusammensetzungen haben aber eine Reihe von Nachteilen. Die Penetration in die Haut ist oft eher gering, so dass das Hautgefühl unangenehm fettig sein kann. Die Fette erreichen durch die äußerst geringe Penetration nur die äußeren Bereiche des 12-20 Hornschichten umfassenden Stratum Corneum. Sobald die aufgebrachten Fette durch den Kontakt mit Wasser und waschaktiven Substanzen abgewaschen werden, verschwindet die Wirkung größtenteils. Auch über die ständige Reibung an Textilien werden die Fette wieder abgetragen. Mit zunehmendem Alter nimmt zusätzlich die Aktivität der Keratinozyten ab, d.h. die Zellteilung bzw. Hautregeneration und somit die Bildung der körpereigenen Hautbarriere nimmt ab.

Damit besteht ein Bedarf an verbesserten pharmazeutischen und kosmetischen Behandlungsmethoden, insbesondere ein Bedarf an Zusammensetzungen, die sowohl die Schutz- und Barrierefunktion der Haut stärken als auch gleichzeitig das Hautgefühl und das Hautbild verbessern, wobei diese Zusammensetzungen keine oder nur vernachlässigbare Nebenwirkungen zeigen sollen. Es besteht insbesondere ein Bedarf an Zusammensetzungen, die tiefer in die Hornschichten eindringen und den Zusammenhalt der Hornzellen im Stratum Corneum stärken und so die Schutzfunktion der Epidermis unterstützen und Beeinträchtigungen der Haut durch Vorbelastungen, wie unter anderem schlecht heilende Wunden, Irritationen, Reizungen, Entzündungen und andere, ausgleichen.

Ausgehend hiervon war es die Aufgabe der vorliegenden Erfindung, eine gut verträgliche Zusammensetzung zur Behandlung der Haut, insbesondere zur Stärkung der Schutz- und Barrierefunktion der Haut, bereitzustellen. Weiterhin sollte sich diese Zusammensetzung topisch anwenden lassen und die Nachteile der aus dem Stand der Technik bekannten Zusammensetzungen überwinden.

Insbesondere war es die Aufgabe der vorliegenden Erfindung, eine wirksame und gut verträgliche Zusammensetzung bereitzustellen, die unterstützend auf die Regeneration der Epidermis wirkt und die Wundheilung positiv beeinflusst. Gleichzeitig sollte die Zusammensetzung gut verarbeitbar sein.

Diese Aufgabe wurde überraschend durch die erfindungsgemäße Zusammensetzung und deren Verwendung gelöst.

Ein erster Aspekt der Erfindung betrifft eine Zusammensetzung zur Behandlung des menschlichen Epithelgewebes, umfassend
(A) wenigstens eine organische Säure mit mindestens zwei funktionellen Gruppen, wobei wenigstens eine funktionelle Gruppe eine Carbonsäure ist, und
(B) ein in Wasser lösliches anorganisches mehrwertiges Salz, wobei die Zusammensetzung einen pH-Wert zwischen 2,0 und 8,0, besonders zwischen pH 3,5 und 5,5, insbesondere zwischen den pH-Werten 4,0 und 5,0 aufweist.

Die erfindungsgemäße Zusammensetzung umfasst bevorzugt ferner
(C) wenigstens eine Aminosäure, ein Aminosäurederivat und/oder beliebige Mischungen davon.

### Organische Säure (A)

Die erfindungsgemäße Zusammensetzung umfasst eine organische Säure mit mindestens zwei funktionellen Gruppen, wobei wenigstens eine funktionelle Gruppe eine Carbonsäure ist.

Der Begriff "funktionelle Gruppe", wie hierin verwendet, beschreibt eine Gruppe umfassend mindestens ein Heteroatom. Generell bestimmen funktionelle Gruppen einer chemischen Verbindung maßgeblich die Stoffeigenschaften und das Reaktionsverhalten der Verbindung. Die Heteroatome der funktionellen Gruppen können unabhängig aus O, N, P oder S ausgewählt werden.

Erfindungsgemäß bevorzugte funktionelle Gruppen können unabhängig ausgewählt sein aus der Gruppe umfassend -COOH, -(C=O)-, -C(=O)-O-, -OH , -SH, -NR2, wobei R wiederum unabhängig ausgewählt ist aus H und Methyl, Ethyl oder Propyl, wobei H bevorzugt ist.

Die organische Säure (A) ist bevorzugt eine Carbonsäure und insbesondere bevorzugt eine mehrwertige Carbonsäure mit pKs-Werten zwischen 1,5 und 10. Besonders bevorzugt ist (A) eine mehrwertige Carbonsäure mit pKs-Werten zwischen 2,5 und 6,0. Die organische Säure (A) kann in bevorzugten Ausführungsformen eine Dicarbonsäure sein.

Die organische Säure (A) ist bevorzugt ausgewählt aus der Gruppe bestehend aus Äpfelsäure, Maleinsäure, Weinsäure, Fumarsäure, Gallussäure, Gluconsäure, Glycolsäure, Mandelsäure, Milchsäure, Bernsteinsäure, Brenztraubensäure, L-(+)-Ascorbinsäure, D-Chinasäure, Citronensäure, Glutarsäure, Malonsäure und/oder Mischungen davon.

Äpfelsäure ist insbesondere bevorzugt. Andere insbesondere bevorzugte Säuren sind Bernsteinsäure, Maleinsäure und Fumarsäure sowie Mischungen davon.

Äpfelsäure (2-Hydroxybernsteinsäure) umfasst erfindungsgemäß rechtsdrehende D- und als linksdrehende L-Äpfelsäure sowie Racemate und beliebige Mischungen von D- und L-Äpfelsäure.

Allgemein können erfindungsgemäß organische Säuren, die als Enantiomere vorkommen, in Ihrer D oder L bzw. R oder S-Konfiguration, als Racemat (1:1 Gemisch der Enantiomere) oder in beliebigen Mischungen eingesetzt werden.

Bevorzugt umfasst die erfindungsgemäße Zusammensetzung 0,001 bis 10 Gew.%, insbesondere 0,01 bis 5% Gew.%, noch stärker bevorzugt 0,1% bis 2,0 Gew.%, organische Säure (A).

Soweit nicht anders angegeben bezieht sich die Angabe "Gew.%" hierin immer auf das Gesamtgewicht der Zusammensetzung.

### In Wasser lösliches anorganisches mehrwertiges Salz (B)

Die erfindungsgemäße Zusammensetzung enthält ein in Wasser lösliches anorganisches mehrwertiges Salz.

Das anorganische Salz weist bevorzugt eine leichte Löslichkeit in Wasser, d.h. eine Löslichkeit größer als 100 g/l bei 20°C, oder eine mäßige Löslichkeit in Wasser, d.h. zwischen 10 und 100g/l bei 20°C, auf. Eine leichte Löslichkeit ist bevorzugt. Bevorzugt liegt das anorganische Salz in der erfindungsgemäßen Zusammensetzung vollständig gelöst vor.

Das anorganische mehrwertige Salz ist bevorzugt zwei- oder dreiwertig. Zweiwertige Salze sind bevorzugt. Die erfindungsgemäße Zusammensetzung kann zusätzlich anorganische einwertige Salze umfassen. In anderen bevorzugten Ausführungsformen umfasst die Zusammensetzung keine einwertigen anorganischen Salze.

Das anorganische mehrwertige Salz umfasst bevorzugt mindestens ein Kation ausgewählt aus der Gruppe bestehend aus Mg2+, Ca2+, Sr2+, Zn2+, Al3+, Fe3+, La3+, oder beliebige Mischungen davon.

Bevorzugt ist das anorganische mehrwertige Salz ein Calciumsalz. In einer weiteren bevorzugten Ausführungsform enthält die erfindungsgemäße Zusammensetzung sowohl ein Calcium- als auch ein Magnesiumsalz.

Das Anion des anorganischen mehrwertigen Salzes leitet sich von einer anorganischen Säure ab. Bevorzugte anorganische Säuren können dabei ausgewählt werden aus der Gruppe umfassend Salzsäure (beispielhafte Salze NaCl, MgCl2, CaCl2), Schwefelsäure (beispielhaftes Salz Magnesiumsulfat (MgSO4)), Kohlensäure (beispielhaftes Salz Natriumhydrogencarbonat (NaHCO3)), Phosphorsäure (beispielhafte Salze Natriumphosphat (Na3PO4) und Calciumphosphat (Ca3(PO4)2)), Borsäure (beispielhaftes Salz Natriumborat (Na2B4O7)) und Phosphinsäure (beispielhaftes Salz Calciumhypophosphit (Ca(H2PO2)2)).

Erfindungsgemäß ist Phosphinsäure insbesondere bevorzugt.

Phosphinsäure, auch Hypophosphorige Säure, ist eine der Säuren des Phosphors und hat die Summenformel H3PO2. Ihre Salze werden Phosphinate oder Hypophosphite genannt. Erfindungsgemäß werden zweiwertige Salze der Phosphinsäure verwendet. Eine zusätzliche Verwendung von einwertigen Salzen ist nicht ausgeschlossen.

Erfindungsgemäß ist als wasserlösliches anorganisches mehrwertiges Salz (B) Calciumhypophosphit (Ca(H2PO2)2) besonders bevorzugt.

Die erfindungsgemäße Zusammensetzung enthält in bevorzugten Ausführungsformen 0,001 bis 30,0 Gew.%, insbesondere 0,01 bis 10 Gew.%, bevorzugt 0,1 bis 2,0 Gew.%, besonders bevorzugt 0,3 bis 1,0 Gew.% in Wasser lösliches anorganisches mehrwertiges Salz.

Die erfindungsgemäße Zusammensetzung enthält in bevorzugten Ausführungsformen 0,001 bis 30,0 Gew.%, insbesondere 0,01 bis 10 Gew.%, bevorzugt 0,1 bis 2,5 Gew.% Calciumhypophosphit.

In einer weiteren bevorzugten Ausführungsform enthält die erfindungsgemäße Zusammensetzung ein Calciumsalz und ein Magnesiumsalz, wobei das Calciumkationen und die Magnesiumkationen in einem Gewichtsverhältnis von 1:2 bis 1:4,5, bevorzugt in einem Gewichtsverhältnis von 1:3,0 bis 1:3,5, vorliegen. Die Verhältnisse liegen im Bereich der Verhältnisse von Meerwasser

Bevorzugt liegt das Calciumsalz dabei in einer Menge von 0,1 bis 2,5 Gew.%, besonders bevorzugt von 0,8 bis 1,5 Gew.%, und das Magnesiumsalz in einer Menge von 0,3 bis 8 Gew.%, bevorzugt von 3,0 bis 4,5 Gew. % vor.

### Zusammensetzung

Die erfindungsgemäße Zusammensetzung weist einen pH-Wert zwischen 2,0 und 8,0 auf.

Der pH-Wert der erfindungsgemäßen Zusammensetzung liegt bevorzugt im Bereich zwischen 3 bis 7, insbesondere bevorzugt im Bereich zwischen 4 bis 6, und besonders bevorzugt im pH-Bereich zwischen 4,0 bis 5,0. Wie bereits beschrieben kann auch der pH-Wert der Zusammensetzung durch pH-Regulatoren eingestellt werden.

Die Zusammensetzung umfasst bevorzugt wenigstens ein in Wasser lösliches Calciumsalz.

Die Zusammensetzung umfasst bevorzugt Glycerin. Glycerin hilft u.a., Feuchtigkeit in der Haut zu binden und kann so helfen trockene und rissige Haut zu verhindern. Glycerin ist für alle Hauttypen geeignet.

Die Zusammensetzung weist bevorzugt eine Viskosität zwischen 100 und 150.000 mPsec auf (jeweils 0-4 Tage nach Herstellung bestimmt).

Alle Viskositätsangaben beruhen auf einer Messung nach DIN 53019-1:2008-09 mit einem Rheometer des Typs Haake RheoStress1 (ThermoFisher Scientific) bei 20 °C und einer Schergeschwindigkeit von 10/s in Platte-Platte Geometrie (Drehkörper PP60 Ti).

Der pH-Wert der Zusammensetzung liegt vorzugsweise im Bereich von 3,0 bis 8,0, insbesondere zwischen 5,0 und 7,0.

Der pH-Wert kann durch pH-Regulatoren eingestellt werden. pH-Regulatoren sind Substanzen, die einen bestimmten pH-Wert-Bereich, vorzugsweise einen Bereich von pH 5,5 bis 8,0, einstellen können.

Beispiele für pH-Regulatoren sind Essigsäure, Acetaten, Milchsäure, Lactaten, Äpfelsäure, Malate, Fumarsäure, Citronensäure, Citrate, Weinsäure, Tartrate, Orthophosphaten, Di-, Tri- und Polyphosphate, Salzsäure, Chloriden, Schwefelsäure, Sulfate, Hydroxiden, Oxide, Adipinsäure, Adipate, Gluconsäure, Gluconate, Phosphorsäure, Calciumcarbonat oder ein Hydrat davon. Ein bevorzugtes Beispiel für einen pH-Regulator, der zugegeben werden kann, wenn ein niedrigerer pH-Wert gewünscht wird, ist Phosphorsäure (H3PO4).

### Aminosäure/Aminosäurederivat (C)

Die erfindungsgemäße Zusammensetzung kann bevorzugt wenigstens eine Aminosäure, ein Aminosäurederivat und/oder beliebige Mischungen davon umfassen.

Die erfindungsgemäße Zusammensetzung kann jede beliebige Aminosäure umfassen. Bevorzugte Aminosäuren sind Arginin, Asparagin, Valin, Serin, Cystein, Tryptophan, Glutamin, Histidin, Methionin, Lysin, Prolin, Leucin und/oder Glycin.

Arginin spielt eine wichtige Rolle bei der Zellteilung sowie der DNA-Synthese. Arginin unterstützt die Hautfeuchtigkeit und wirkt regenerierend auf beschädigte Hautzellen. Asparagin ist an der Kollagenbildung beteiligt und kräftigt die Hautoberfläche durch verbesserte Stabilität, Spannkraft und Festigkeit.

Valin wirkt feuchtigkeitsspendend und hat eine antioxidative Wirkung.

Serin wirkt feuchtigkeitsspendend, hautberuhigend, entzündungshemmend, unterstützt die Zellregeneration und hat eine antioxidative Wirkung.

Serin wirkt feuchtigkeitsspendend. Indem es die Feuchtigkeit der Haut verbessert, trägt Serin dazu bei, die Hautelastizität zu steigern, was zu einem strafferen Hautbild führt.

Tryptophan stärkt die hauteigene Feuchtigkeitsbarriere und sorgt für einen strahlenden Teint. Tryptophan wirkt regulierend auf die Ölproduktion der Haut und mindert Pigmentflecken und Rötungen.

Glutamin fördert die Bildung von Hautzellen und verhindert bzw. verlangsamt den Abbau von Gewebestrukturen und Hautalterung.

Histidin wirkt auf die Haut beruhigend und hat antioxidantische Eigenschaften.

Methionin wirkt durch seine antioxidantische Eigenschaften schützend auf die Haut.

Lysin ist an der Kollagensynthese beteiligt und kräftigt die Hautoberfläche durch verbesserte Stabilität, Spannkraft und Festigkeit. Lysin spielt auch bei der Reparatur von Hautschäden eine tragende Rolle.

Prolin, Leucin und Glycin vermindern feine Fältchen und Falten. Prolin ist ebenso wie Glycin an der Kollagenproduktion beteiligt. Glycin ist an der Bildung von Elastin und Kollagen beteiligt. Es sorgt nicht nur für die Elastizität und Festigkeit der Haut, sondern beugt auch Gewebeabbau vor. Zusammen mindern Prolin, Leucin und Glycin die Entstehung von Krähenfüßen.

Aminosäurederivate im Sinne der vorliegenden Erfindung sind chemische Verbindungen, die aus Aminosäuren abgeleitet werden. Aminosäurederivate entstehen, wenn die Aminosäurestruktur durch chemische Reaktionen verändert wird, wie z.B. durch die Modifikation der funktionellen Gruppen der Aminosäuren (Aminogruppe oder Carbonsäuregruppe) oder durch die Einführung zusätzlicher Substituenten. Beispielhafte Modifikationen umfassen Hydroxylierung, Carboxylierung, Phosphorylierung, Methylierung und/oder Acetylierung.

Aminosäurederivate, welche in der Pharmazie und/oder Kosmetik eine Rolle spielen, sind dem Fachmann bekannt und umfassen Neurotransmitter (z.B. Dopamin (abgeleitet von Tyrosin), Serotonin (abgeleitet von Tryptophan), GABA (Gamma-Aminobuttersäure (abgeleitet von Glutamat), Hormone (z.B. Thyroxin (abgeleitet von Tyrosin), Adrenalin (abgeleitet aus Tyrosin)), Alkaloid (z.B. Histamin (abgeleitet von Histidin)), Fettsäurederivate (z.B. Sphingosin (abgeleitet aus Serin)), Ceramide (Lipidverbindungen aus sphingolipidähnlichen Aminosäurederivaten), Natrium-PCA (Salz der Pyrrolidoncarbonsäure (abgeleitet aus Glutaminsäure)), Glutathion (Tripeptid, aus Glutaminsäure, Cystein und Glycin), aminosäurebasierte Tenside (z.B. Sodium Cocoyl Glutamate) und/oder Dimethylglycin oder ein Dimethylglycinat.

Die erfindungsgemäße Zusammensetzung umfasst bevorzugt 0,0001 bis 10 Gew.-%, insbesondere bevorzugt 0,01 bis 5 Gew.-% eine Aminosäure, ein Aminosäurederivat und/oder beliebige Mischungen davon.

Die erfindungsgemäße Zusammensetzung umfasst bevorzugt das Aminosäurederivat (C) Dimethylglycin oder ein Dimethylglycinat.

Dimethylglycin (N,N-Dimethylglycin) kommt in Pflanzen, Tieren und dem Menschen vor, wobei es im Menschen nur in sehr geringen Mengen gebildet wird. Es entsteht bei einer mehrstufigen Biosynthese von Glycin aus Cholin als Zwischenprodukt durch Transaminierung von Betain mit Betain-Homocystein-Methylase.

N,N-Dimethylglycin, auch (Dimethylamino)essigsäure, wird durch die folgende chemische Formel 1 dargestellt:

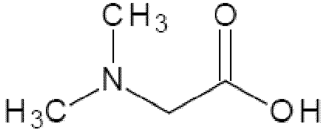

Erfindungsgemäß ist nicht nur der Einsatz von Dimethylglycin, sondern auch der von dessen Salzen, Solvaten und Hydraten. Dabei handelt es sich bevorzugt um pharmazeutisch bzw. kosmetisch annehmbare Salze von Dimethylglycin. Das Salz ist besonders bevorzugt ein wasserlösliches Salz mit einer Löslichkeit in Wasser von mindestens 10 g/l bei 20°C.

In einer bevorzugten Aufführungsform ist das Salz von Dimethylglycin ein Alkali-, Erdalkali- oder Ammoniumsalz von Dimethylglycin.

Beispiele sind Natrium-, Kalium-, Calcium-, Magnesium- und Ammoniumsalze. Bei den Ammoniumsalzen trägt das Ammoniumkation eine bis vier Alkylgruppen mit jeweils unabhängig voneinander 1 bis 4 Kohlenstoffatomen. Bevorzugt sind das Natrium- und Kaliumsalz von Dimethylglycin, insbesondere das Natriumsalz von Dimethylglycin, nämlich Natrium-dimethylglycinat (Natrium-N,N-dimethylglycinat).

In einer alternativ bevorzugten Ausführungsform kann das Salz von Dimethylglycin das Salz einer anorganischen Säure mit Dimethylglycin sein.

Beispiele für Salze von Dimethylglycin mit einer anorganischen Säure sind das Hydrochlorid, Hydrobromid, Hydroiodid, Hydrogensulfat, Sulfat, Hydrogensulfit, Sulfit, Hydrogencarbonat, Carbonat, Monophosphat, Diphosphat und Triphosphat von Dimethylglycin sowie Mischungen daraus. Insbesondere bevorzugt ist das Hydrochlorid von Dimethylglycin, nämlich Dimethylglycin-hydrochlorid (N,N-Dimethylglycin-hydrochlorid).

Erfindungsgemäß bevorzugt ist das Dimethylglycin und/oder Salz von Dimethylglycin ausgewählt aus der Gruppe bestehend aus Dimethylglycin, Natrium-dimethylglycinat und Dimethylglycin-hydrochlorid.

Erfindungsgemäß bevorzugt ist das Dimethylglycin und/oder Salz von Dimethylglycin ausgewählt aus der Gruppe bestehend aus Dimethylglycin, Natrium-dimethylglycinat und Dimethylglycin-hydrochlorid.

Die erfindungsgemäße Zusammensetzung umfasst bevorzugt 0,0001 bis 10 Gew.-%, insbesondere bevorzugt 0,01 bis 5 Gew.-%, noch stärker bevorzugt 0,1 bis 2,0 Gew.-% Dimethylglycin oder eine Dimethylglycinat oder beliebige Mischungen davon.

### Weitere Bestandteile der erfindungsgemäßen Zusammensetzung

Die erfindungsgemäße Zusammensetzung kann ferner wenigstens einen weiteren Wirkstoff und/oder Additiv umfassen.

Antibakterielle Substanzen sind unter anderem bevorzugt. Zink ist eine erfindungsgemäß bevorzugte Substanz mit antibakterieller Wirkung. Die erfindungsgemäße Zusammensetzung kann Zink insbesondere bevorzugt in einer Menge von 0,01-30,0 Gew.-%, bevorzugt 0,1-5,0 Gew.-%, jeweils bezogen auf das Gesamtgewicht der Zusammensetzung umfassen.

Vorzugsweise ist der Wirkstoff ausgewählt aus der Gruppe bestehend aus Biotin, Menthol, Taurin, Ectoin, Ubichinon-10, Echinacea, Panthenol, Nicotinsäureamid, Tocopherol, Tocopherolacetat, Harnstoff und Salz der Pyrrolidoncarbonsäure und beliebigen Mischungen davon.

Biotin, welches auch als Vitamin B7 oder Vitamin H bezeichnet wird, ist ein wasserlösliches Vitamin aus dem B-Komplex. Biotin kann die Haut erfindungsgemäß stärken. In der erfindungsgemäßen Zusammensetzung kann Biotin in einer Menge von 0,001-10,0 Gew.-%, bevorzugt 0,005-1,0 Gew.-% enthalten sein, jeweils bezogen auf das Gesamtgewicht der Zusammensetzung.

Menthol ist ein monocyclischer Monoterpenalkohol und kann der erfindungsgemäßen Zusammensetzung als die Durchblutung stimulierender Wirkstoff zugesetzt werden. Zudem kann Menthol eine erfrischende sensorische Stimulierung der Haut bewirken.

Taurin oder 2-Aminoethansulfonsäure wirkt erfindungsgemäß als Antioxidans ebenfalls weiter stabilisierend auf die Haut.

Ectoin ist eine zyklische Aminosäure und liegt in wässriger Lösung als mesomeriestabilisiertes Zwitterion vor. Ectoin wirkt befeuchtend und stabilisiert erfindungsgemäß die natürliche Struktur der Haut. Zudem hat sich gezeigt, dass Ectoin vor UV-Strahlung schützt und bei der Behandlung entzündlicher Krankheiten hilfreich sein kann.

Ubichinon-10 (Q10 oder Coenzym Qio) ist ein Chinon-Derivat. Das zum Ubichinon-Pool gehörende Q10 gilt als Antioxidans und wirkt erfindungsgemäß stabilisierend auf die Haut.

Echinacea wirkt erfindungsgemäß beruhigend auf die Haut und lindert auftretenden Juckreiz sowie Spannungen. Zudem kann Echinacea die Blutzirkulation der Haut anregen.

Panthenol (auch Dexpanthenol) spielt bei der Wundheilung der Haut, insbesondere der Epidermis eine Rolle. Nach Ausnahme durch die Haut wird es zu Pantothensäure umgewandelt, die bei der Neubildung von Zellen eine tragende Rolle spielt. Daneben erhöht Panthenol das Feuchthaltevermögen der Haut, was sie pflegt und ihre Elastizität verbessert. Ferner werden entzündungshemmende und juckreizlindernde Wirkungen beschrieben.

Nicotinsäureamid (auch Niacinamid und Nicotinamid) ist das Amid der Nicotinsäure und wird auch als Vitamin B3 bezeichnet. Neben anderen Eigenschaften wie beispielweise einer Verringerung von oxidativem Stress hat das Niacinamid erfindungsgemäß eine Hautschützende Wirkung.

Tocopherol, welches auch als Vitamin E bezeichnet wird, ist eine fettlösliche Substanz mit die Haut stärkender antioxidativer Wirkung.

Tocopherolacetat zeigt antioxidative Eigenschaften und wirkt erfindungsgemäß stabilisierend auf die Haut.

Harnstoff zieht Wasser an und bindet es in der Haut. Dies hilft, die Haut mit Feuchtigkeit zu versorgen und ihre Hydratation zu verbessern. Diese Eigenschaft ist besonders vorteilhaft bei trockener Haut. Die Haut wird insbesondere widerstandsfähiger gegenüber äußeren Einflüssen wie Kälte, Wind oder trockener Luft. In höheren Konzentrationen (typischerweise 5-10%) wirkt Harnstoff ferner keratolytisch, was bedeutet, dass es abgestorbene Hautzellen auf der Hautoberfläche sanft entfernt. Dies kann dazu beitragen, die Haut zu glätten und das Erscheinungsbild von rauer oder schuppiger Haut zu verbessern. Aufgrund seiner feuchtigkeitsspendenden und exfolierenden Eigenschaften kann Harnstoff auch in Anti-Aging-Produkten eingesetzt werden. Es hilft, feine Linien und Falten zu mildern, indem es die Hautstruktur verbessert. Harnstoff hat auch entzündungshemmende Eigenschaften und kann bei der Beruhigung von gereizter Haut helfen. Deshalb wird er häufig in Produkten für empfindliche Haut oder bei Hauterkrankungen wie Ekzemen oder Psoriasis verwendet.

Pyrrolidoncarbonsäure bzw Salze der Pyrrolidoncarbonsäure spielen in der Hautpflege eine wichtige Rolle. Das Natriumsalz der Pyrrolidoncarbonsäure kommt in der menschlichen Haut vor und schützt durch seine Fähigkeit, einen Hydratationsgrad in der obersten Schicht der Epidermis aufrechtzuerhalten vor Austrocknung. Durch Anregung des epidermalen Differenzierungsprozesses kann es die schützende Funktion der Hautbarriere verstärken. Erfindungsgemäß sind das Natrium- und Zink-Salz der Pyrrolidoncarbonsäure insbesondere bevorzugt.

In einer bevorzugten Ausführungsform der Erfindung enthält die erfindungsgemäße Zusammensetzung mindestens einen weiteren Wirkstoff ausgewählt aus Menthol, Biotin, Niacinamid, Panthenol, Ectoin, Ubichinon-10, Taurin, Pantolacton, Echinacea, Tocopherol, Tocopherolacetat, Harnstoff, Salz der Pyrrolidoncarbonsäure und beliebige Mischungen davon jeweils in einem Anteil von 0,001 Gew.-% bis 10,0 Gew. -%, mehr bevorzugt 0,005 Gew.-% bis 7,50 Gew.-%, noch mehr bevorzugt von 0,01 Gew.-% bis 5,0 Gew.-%, insbesondere von 0,1 Gew.-% bis 3,0 Gew.-%, bezogen auf das Gesamtgewicht der Zusammensetzung.

In einer Ausführungsform umfasst die Zusammensetzung ein Tensid. Das Tensid kann ein anionisches, nichtionisches, kationisches oder zwitterionisches Tensid sein. Bevorzugt ist das Tensid ein anionisches oder nichtionisches Tensid, insbesondere ein möglichst mildes (d.h. besonders hautverträgliches) anionisches oder nichtionisches Tensid. Nichtionische Tenside werden dabei erfindungsgemäß insbesondere wegen ihrer sehr guten Emulgationseigenschaften sowie ihrer ausgezeichneten Hautpflegeeigenschaften eingesetzt. Anionische Tenside sind bevorzugt, weil sie eine besonders hohe Reinigungsleistung aufweisen. Die bevorzugten Tenside der vorliegenden Erfindung sind u.a. in dem Buch "Surfactants and interfacial phenomena1 ‴, von Milton Rosen und Joy Kunjappu, John Wiley & Sons, Ine. -Verlag, 2012, 4. Auflage beschrieben.

In einer bevorzugten Ausführungsform ist das Tensid ein anionisches Tensid ausgewählt aus Alkylsulfonaten, Alkylsulfaten, Alkylethersulfaten, Alkylphosphaten, Alkylsarkosinaten, Alkyltauraten, Aminosäure-Tensiden und Mischungen davon. Besonders bevorzugt ist das Tensid ausgewählt aus Alkylsulfaten, Alkylsarkosinaten, Alkyltauraten, Alkylglutamat, wie Natriumcocoylglutamat/Dinatriumcocoylglutamat, Alkylgycinat, Alkylalaninat, wie Natriumcocoylalaninat und Mischungen davon. Ebenso bevorzugt wegen ihrer Reinigungsleistung sind Fettalkoholpolyglycerolethersulfate, Monoglyceridsulfate, Mono- und/oder Dialkylsulfosuccinate, Fettsäureisethionate, und oc-Olefinsulfonate.

In einer Ausführungsform ist das Tensid ein nichtionisches Tensid (auch als nichtionischer Emulgator bezeichnet). Nicht-einschränkende Beispiele umfassen Glycerinfettsäureester, Polyoxyethylenether eines oder mehrerer Fettalkohole, alkoxylierte Fettsäurealkylester, Polyglycerinether von Fettalkoholen, Polyglycerinester von Fettsäuren, Polyethylenglykol - und/oder Polypropylenglykolether, Fettsäureamide, Alkylphenolpolyglycolether, Aminoxide und Alkylpolyglucoside.

In einer Ausführungsform ist das Tensid ausgewählt aus der Gruppe der Glycerinfettsäureester, Polyoxyethylenether eines oder mehrerer Fettalkohole, Polyglycerinether von Fettalkoholen, Polyglycerinester von Fettsäuren und deren Mischungen.

In einer Ausführungsform umfasst das Tensid ein kationisches Tensid, wie beispielsweise ein quaternäres Tensid. Quaternäre Tenside enthalten mindestens ein N-Atom, das mit 4 Alkyl oder Arylgruppen kovalent verbunden ist. Dies führt, unabhängig vom pH-Wert, zu einer positiven Ladung. Vorteilhaft sind Alkylbetain, Alkylamidopropylbetain und Alkylamidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, hmdazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

In einer bevorzugten Ausführungsform ist die erfindungsgemäße Zusammensetzung eine Ölin-Wasser-Emulsion (O/W), in der die vorstehend beschriebenen Tensidkomponenten (auch als Emulgatorkomponenten bezeichnet) einen HLB-Wert von 8-40, bevorzugt von 8-22, besonders bevorzugt von 8-18 aufweisen. In einer anderen bevorzugten Ausführungsform ist die erfindungsgemäße Zusammensetzung eine Wasser-in-ÖI-Emulsion (W/O), in der die vorstehend beschriebenen Tensidkomponenten (auch als Emulgatorkomponenten bezeichent) einen HLB-Wert von 3-8, bevorzugt von 4-5 aufweisen. Das Berechnungsverfahren für den kombinierten HLB-Wert ist bekannt und kann als Griffin- oder Davies-Verfahren bezeichnet werden (Ullmann's Encyclopedia of Industrial Chemistry, Teil 9, Emulsion, 2005; Surfactant Application Encyclopedia, Liu Cheng, Beijing Industry Press, 1997).

Additive können ausgewählt sein aus der Gruppe bestehend aus Rückfettern, Konservierungsmitteln, Stabilisatoren, Duftstoffen, Antioxidantien, Rheologiemodifikatoren, Verdickungsmitteln, Pflegemitteln, Farbstoffen, Perlganzmitteln, Aufhellungsmitteln, Lösungsmitteln, Sonnenschutzmittel und Kombinationen hiervon.

Bevorzugt sind Additive, die üblicherweise in Emulsionen zur Behandlung der Haut eingesetzt werden. Das mindestens ein Additiv kann in einem Anteil von 0,01 Gew.-% bis 12,0 Gew.-%, mehr bevorzugt von 0,25 Gew.-% bis 10,0 Gew.-%, insbesondere von 1,0 bis 7,0 Gew.-% vorliegen.

Rückfetter, auch Rückfettungsmittel oder Überfettungsmittel genannt, sind lipophile Substanzen, die eine störende Auswirkung auf die epidermale Barrierefunktion verhindern können. Beispiele für Rückfetter sind Wollwachs, Squalan, flüssiges Paraffin, pflanzliche Öle, Silikone und Cetylpalmitat.

Konservierungsmittel sind Substanzen, die zur Konservierung verwendet werden, indem sie die Zusammensetzung zersetzende Mikroorganismen abtöten und/oder deren Wachstum hemmen. Vorzugsweise können die Konservierungsmittel ausgewählt sein aus der Gruppe bestehend aus Benzoesäure, Benzoesäurederivaten, Sorbinsäure, Sorbinsäurederivaten, Salicylsäure, Salicylsäurederivaten, Phenoxyethanol, Parabenen und Kombinationen hiervon. In einer bevorzugten Ausführungsform werden Natriumbenzoat und/oder Kaliumsorbat als Konservierungsmittel in der erfindungsgemäßen Zusammensetzung eingesetzt. Natriumbenzoat setzt in leicht saurem Milieu Benzoesäure und Kaliumsorbat Sorbinsäure frei. Beiden Säuren wird eine antimikrobielle Wirkung zugeschrieben.

Stabilisatoren können lichtempfindliche Komponenten gegenüber Strahlung schützen und sind bevorzugt UV- Absorber wie beispielsweise Benzophenonderivate.

Die Zugabe von Duftstoffen kann für einen angenehmen Geruch der Zusammensetzung sorgen. Beispiele sind die dem Fachmann bekannten Parfums.

Ein Antioxidans oder Antioxidationsmittel ist eine chemische Verbindung, die eine Oxidation anderer Komponenten in der erfindungsgemäßen Zusammensetzung verlangsamt oder gänzlich verhindert. Als Antioxidantien kommen beispielsweise Zitronensäure, Ascorbinsäure und Butylhydroxyanisol in Frage.

Rheologiemodifikatoren und Verdickungsmittel können dabei helfen, die Applikationseigenschaften der erfindungsgemäßen Zusammensetzung zu verbessern. Als Rheologiemodifikator und Verdickungsmittel kann die Zugabe von Kochsalz (Natriumchlorid) in Betracht gezogen werden. Durch die Zugabe von Kochsalz kann die Fließfähigkeit der erfindungsgemäßen Zusammensetzung in gewissen Grenzen beeinflusst und auf das nötige Maß eingestellt werden. Als Verdicker können zusätzlich natürlich vorkommende Gelbildner eingesetzt werden, die bevorzugt ausgewählt sind aus Agar, Xanthan Gum, Cellulose und oder Cellulose-Derivaten oder Alginsäure.

Im Rahmen der Anmeldung sollen unter Pflegemitteln Substanzen verstanden werden, welche die Haut pflegen. Hydrolysiertes Weizenprotein, Shea Butter, Harnstoff und Allantoin wirken pflegend auf die Haut.

Hydrolysiertes Weizenprotein hat hauptsächlich feuchtigkeitsspendende Eigenschaften.

Shea Butter zeigt eine intensive Pflegewirkung und versorgt die Haut mit essenziellen Fettsäuren. Shea Butter eignet sich besonders für trockene und raue Hautpartien und hinterlässt ein angenehmes Hautgefühl.

Harnstoff wirkt feuchtigkeitsspendend und hilft, raue Haut zu glätten.

Allantoin fördert die Hautregeneration. Es unterstützt die Bildung neuer Hautzellen und unterstützt die Heilung kleinerer Verletzungen oder Risse.

Farbstoffe werden optional dazu verwendet, der erfindungsgemäßen Zusammensetzung eine charakteristische Farbe zu verleihen, so dass diese leicht von anderen Produkten unterschieden werden kann.

Als Lösungsmittel kann ein für den Fachmann auf diesem Gebiet gängiges Lösungsmittel oder Lösungsmittelgemisch eingesetzt werden. Bevorzugte Lösungsmittel sind Ethanol oder Butylenglykol, insbesondere 1,4-Butylenglykol, Propylenglykol und Isopropylalkohol. Bevorzugt ist Ethanol. Diese Lösungsmittel können vorzugsweise in einer Menge von 0,1 bis 70 Gew.% in der erfindungsgemäßen Zusammensetzung enthalten sein. Ganz besonders bevorzugt sind sie in einer Menge von 0,1 bis weniger als 5,0 Gew.% enthalten. Außerdem helfen Lösungsmittel bei der Penetration der Wirkstoffe und verstärken somit deren Wirksamkeit.

Geeignete Sonnenschutzmittel sind UVA-Filtersubstanzen und/oder UVB-Filtersubstanzen und/oder anorganische Pigmente.

Vorteilhaft können erfindungsgemäße Emulsionen Substanzen enthalten, die UV-Strahlung im UVB- Bereich absorbieren, wobei die Gesamtmenge der Filtersubstanzen z. B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Emulsion beträgt.

Die UVB-Filter können öllöslich oder wasserlöslich sein. Als öllösliche Substanzen sind z. B. zu nennen: 3-Benzylidencampher und dessen Derivate, z.B. 3-(4-Methylbenzyliden)campher, 4-Aminobenzoesäurederivate, vorzugsweise 4-(Dimethylamino)-benzoesäure(2-ethylhexyl)ester, 4-(Dimethylamino)benzoesäureamylester, Ester der Zimtsäure, vorzugsweise 4-Methoxyzimtsäure(2-ethylhexyl)ester, 4-Metboxyzimtsäureisopentylester; Ester der Salicylsäure, vorzugsweise Salicylsäure(2-ethylhexyl)ester, Salicylsäure.(4-isopropylbenzyl)ester, Salicylsäurehomomenthylester, Derivate des Benzophenons, vorzugsweise 2-Hydroxy-4-methoxybenzophenon, 2-Hydroxy-4-methoxy-4'-methylbenzophenon, 2,2'- Dihydroxy-4-metboxybenzophenon, Ester der Benzalmalonsäure, vorzugsweise 4-Methoxybenzalmalonsäuredi(2- ethylhexyl)ester, 2,4,6- Trianilino-(p-carbo-2'-ethyl-r-hexyloxy)-I,3,5-triazin.

Als wasserlösliche Substanzen sind vorteilhaft: 2-Phenylbenzimidazol-5-sulfonsäure und deren Salze, z. B. Natrium-, Kalium- oder Triethanolammonium-Salze, Sulfonsäure-Derivate von Benzophenonen, vorzugsweise 2-Hydroxy-4- methoxybenzophenon-5-sulfonsäure und ihre Salze; Sulfonsaure-Derivate des 3-Benzylidencamphers, wie z.B. 4-(Oxo-3-bornylidenmethyl)benzolsulfonsäure, 2-Methyl-5-(2-oxo-3-bomylidenmethyl)sulfonsäure und ihre Salze.

Die erfindungsgemäßen Emulsionen können auch UVA-Filter enthalten. Bei solchen Filtersubstanzen handelt es sich vorzugsweise um Derivate des Dibenzoylmethans, insbesondere um I-(4'-tert.Butylphenyl)-3-(4'-methoxyphenyl)-propan-I,3-dion und um 1-Phenyl-3-(4-isopropylphenyl)propan-I,3-dion. Auch Emulsionen, die Kombinationen der genannten Filter enthalten, sind Gegenstand der Erfindung. Es können die gleichen Mengen an UVA-Filtersubstanzen verwendet werden, welche für UVB -Filtersubstanzen genannt wurden.

Die erfindungsgemäßen Emulsionen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon. Besonders bevorzugt handelt es sich um Pigmente auf der Basis von Titandioxid. Es können die für die vorstehenden Kombinationen genannten Mengen verwendet werden.

### Anwendung der Zusammensetzung

Die erfindungsgemäße Zusammensetzung ist zur Behandlung des menschlichen Epithelgewebes geeignet.

Entsprechend umfasst die Erfindung die erfindungsgemäße Zusammensetzung in einem Verfahren zur Behandlung und/Vorbeugung von Schäden des menschlichen Epithelgewebes.

Das Epithelgewebe zählt zu den vier Grundgewebearten, zu denen außerdem noch das Muskel-, Nerven, und Bindegewebe gehören. Es handelt sich um Zellverbände, welche Oberflächen bedecken und auskleiden und dabei unterschiedlichste Funktionen erfüllen.

Eine grobe Unterscheidung kann anhand der Lage und Funktion des Gewebes getroffen werden. Das Drüsenepithel als Teil des Drüsengewebes und das Oberflächenepithel, welches aufgrund verschiedener Anordnungen der Zellen im Verband nochmals unterteilt wird in: einschichtiges Epithel, mehrreihiges Epithel, mehrschichtiges Epithel und Übergangsepithel (Urothel).

Erfindungsgemäß ist die Behandlung von Oberflächenepithel bevorzugt.

Besonders bevorzugt ist dabei die Behandlung von mehrschichtigem Epithel. Mehrschichtiges Epithel kann verhornt oder unverhornt sein. Insbesondere bevorzugt ist mehrschichtiges, verhorntes oder unverhorntes Plattenepithel. Ein Plattenepithel ist ein Epithel, dessen oberste Zellschicht aus flachen, miteinander verbundenen Zellen besteht.

Besonders bevorzugt ist die Behandlung der Epidermis, dem Epithel der Haut, histologisch mehrschichtigem verhorntem Plattenepithel. Entsprechend stellen die Behandlung, Pflege und/oder Verbeugung von Schäden der Haut bevorzugte Ausführungsformen dar.

Eine andere bevorzugte Ausführungsform umfasst die Behandlung von unverhorntem mehrschichtigen Plattenepithel, insbesondere der Mundhöhle, der Speiseröhre, des Analkanals, der Vagina und der Bindehaut des Auges.

Die erfindungsgemäße Zusammensetzung wird topisch angewendet. "Topisch" beschreibt eine äußerliche, in der Regel äußerlich örtliche, Anwendung.

Erfindungsgemäße Zusammensetzungen sind bevorzugt Hautpflegeprodukte (d.h. Behandlungsmittel wie Kuren, Lotionen, Duschbäder, Tages- oder Nachtcreme, Gesichtscreme, Gesichtsfluid, Gesichtsmaske, Hautschutzcreme, Reinigungsmittel, Sonnenschutzlotionen, desodorierende Zusammensetzungen, Rasiercreme, After Shave-Balsam, und/oder ein Tonikum) und keine (reinen) Styling- Produkte. Mit anderen Worten liegt bei den topischen Zusammensetzungen der Erfindung der Fokus auf der medizinischen/pharmazeutischen oder kosmetischen Behandlung der Haut.

Die vorliegende Erfindung bezieht sich weiterhin auf die Verwendung der erfindungsgemäßen Zusammensetzung zur Behandlung der Haut. Die Haut ist erfindungsgemäß besonders bevorzugt die Körperhaut, einschließlich des Oberkörpers und des Unterkörpers, sowie einschließlich der Hände, Arme, Beine und Füße, und/oder die Gesichtshaut, einschließlich des Halses und des Nackens. Die Haut umfasst bevorzugt gerade nicht die Kopfhaut, d.h. die Haut unter dem Haupthaar.

In einer bevorzugten Ausführungsform betrifft die Behandlung der Haut die rein optisch-ästhetische Verbesserung, wie die Schaffung eines glatteren und schlicht schöneren Hautbilds. Erfindungsgemäß werden aber besonders bevorzugt auch darüber hinaus gehende kosmetische/medizinische Effekte erzielt, wie eine verbesserte Schutzfunktion und eine gestärkte Barriereeigenschaft. Zudem wird die Wundheilung verbessert.

Anders ausgedrückt führt die erfindungsgemäße Verwendung zu einer deutlichen Verbesserung der epidermalen Barrierefunktionen und der epidermalen Barriereintegrität, zur Verbesserung des Erscheinungsbildes der Haut und der Erhöhung des Feuchtigkeitsgehalts der Haut. Das geht einher mit einer Steigerung der Kohäsion des Stratum corneum und der Homöostase der Hautbarriere und resultiert schließlich in einem verbesserten Schutz vor Infektionen (mikrobiellen Erkrankungen).

Bei medizinischer Verwendung ist erfindungsgemäß sowohl die therapeutische als auch die prophylaktische Behandlung von Hauterkrankungen umfasst. Bei den Erkrankungen handelt es sich bevorzugt um mikrobielle Hautinfektionen, Hautentzündungen, raue Haut, trockene Haut, Hautirritationen, Juckreiz, Pruritus, Allergien, Psoriasis, psoriatische Arthritis, Ekzeme, Scleroderma, atopische Dermatitis, Kontaktdermatitis, systemischen Lupus erythematosus, Akne und Anfälligkeit gegenüber Kontaktallergien.

Ein weiterer Aspekt der Erfindung betrifft die erfindungsgemäße Zusammensetzung zur Verwendung bei der Behandlung und/ oder Vorbeugung von Schuppenflechte (Psoriasis), Hautekzemen, atopische Dermatitis, Neurodermitis, Hautläsionen und/oder Atrophie.

Ein noch weiterer Aspekt der Erfindung betrifft die erfindungsgemäße Zusammensetzung zur Verwendung bei der Behandlung und/ oder Vorbeugung von Wunden und/oder Narben und/oder Lichen Sclerosus.

Die nicht-therapeutische (d.h. rein kosmetische) Verwendung umfasst insbesondere die Behandlung von kosmetischen Indikationen der Haut, insbesondere ausgewählt aus rauer Haut, trockener Haut, sensibler Haut, Hautirritationen, Juckreiz und Pruritus, sowie die Vorbeugung von Hautinfektionen und die Verminderung der Anfälligkeit gegenüber Kontaktallergien. Die erfindungsgemäßen Emulsionen sind insbesondere wirksam in der Verbesserung der epidermalen Barrierefunktionen und der epidermalen Barriereintegrität, bei der Förderung der Wundheilung, in der Behandlung und/oder Prophylaxe von entzündlichen Zuständen der Haut, in der Behandlung und/oder Prophylaxe von Störungen, die mit einer mangelnden Regeneration der Epidermis verbunden sind, sowie in der Verbesserung des Erscheinungsbildes der Haut, der Erhöhung des Feuchtigkeitsgehalts der Haut, der Förderung des natürlichen Hautschutzes und/oder der Vitalisierung der Haut.

Ein letzter Aspekt der vorliegenden Erfindung betrifft eine Zusammensetzung zur Behandlung des menschlichen Epithelgewebes umfassend
(A) 0,01 bis 5 Gew.-% Äpfelsäure,
(B) 0,01 bis 4 Gew.-% Calciumhypophosphit,
(C) 0,01 bis 5 Gew.-% Dimethylglycin und/oder ein Salz davon,
wobei die Zusammensetzung einen pH-Wert zwischen 4,0 und 6,0 aufweist.

Bevorzugt handelt es sich bei dieser Zusammensetzung um ein Hautpflegeprodukt, welches insbesondere zur topischen Anwendung ausgestaltet ist.

### Beispiele

In den folgenden beispielhaften Zusammensetzungen entsprechen die Mengenangaben jeweils dem Gewichtsanteil in % an der fertigen Zusammensetzung.

Der pH-Wert der beispielhaften Zusammensetzungen liegt zwischen 2,0 und 8,0 und kann auf erfindungsgemäß bevorzugte Bereiche von 3,0 bis 7, insbesondere zwischen 4 bis 6, und besonders bevorzugt zwischen 4,0 bis 5,0 eingestellt werden.

### Beispiel 1: Hautpflegecremes

| | Hautpflegecreme Nr. | | | |
|---|---|---|---|---|
| | 1A | 1B | 1C | 1D |
| Wasser | ad 100 | ad 100 | ad 100 | ad 100 |
| Glycerin | 5 | 5 | 5 | 5 |
| Cetylalkohol | 2 | 2 | 2 | 2 |
| Glycerylstearat | 1,5 | 1,5 | 1,5 | 1,5 |
| Squalan | 1,5 | 1,5 | 1,5 | 1,5 |
| Cetearylethylhexanoat | 1 | 1 | 1 | 1 |
| Octyldodecanol | 3 | 3 | 3 | 3 |
| Oleylerucat | 1,5 | 1,5 | 1,5 | 1,5 |
| Avocadoöl | 0,5 | 0,5 | 0,5 | 0,5 |
| Ethyllinoleat | 1,5 | 1,5 | 1,5 | 1,5 |
| PEG(40)stearat | 2 | 2 | 2 | 2 |
| Phenoxyethanol | 0,5 | 0,5 | 0,5 | 0,5 |
| Tocopherol | 0,5 | 0,5 | 0,5 | 0,5 |
| Calciumhypophosphit | 1 | 1 | 1 | 1 |
| Phytosterole | 0,5 | 0,5 | 0,5 | 0,5 |
| Pflanzliches Öl | 0,5 | 0,5 | 0,5 | 0,5 |
| Sorbitantristearat | 0,5 | 0,5 | 0,5 | 0,5 |
| Olivenöl | 0,5 | 0,5 | 0,5 | 0,5 |
| Natrium-N,N-dimethylglycinat | 1 | 1 | 1 | 1 |
| Äpfelsäure | 0,5 | | | |
| Bernsteinsäure | | 0,5 | | |
| Fumarsäure | | | 0,5 | |
| Maleinsäure | | | | 0,5 |

### Beispiel 2: Hautpflegecreme

| Wasser | ad 100 |
|---|---|
| Glycerin | 5 |
| Cetylalkohol | 2 |
| Glycerylstearat | 1,5 |
| Squalan | 1,5 |
| Cetearylethylhexanoat | 1 |
| Octyldodecanol | 3 |
| Oleylerucat | 1,5 |
| Avocadoöl | 0,5 |
| Ethyllinoleat | 1,5 |
| PEG(40)stearat | 2 |
| Phenoxyethanol | 0,5 |
| Tocopherol | 0,5 |
| Calciumhypophosphit | 1 |
| Phytosterole | 0,5 |
| Pflanzliches Öl | 0,5 |
| Sorbitantristearat | 0,5 |
| Olivenöl | 0,5 |
| Natrium-N,N-dimethylglycinat | 1 |
| Äpfelsäure | 0,5 |

### Beispiel 3: Hautpflegecreme

| Wasser | ad 100 |
|---|---|
| Decyloleat | 7 |
| Isopropylmyristat | 6 |
| Hexyldecanol | 4 |
| Hexyldecyllaurat | 3,5 |
| Distelöl | 5 |
| Glycerin | 4 |
| Sorbitan-Isostearat | 2 |
| Polyglycerin-3 Polyricinoleat | 3 |
| Cera Alba | 2,5 |
| Zinkstearat | 1 |
| Benzylalkohol | 0,5 |
| Magnesiumsulfat | 0,4 |
| Echinacea Purpurea Wurzelextrakt | 0,1 |
| Tocopherol | 0,5 |
| Äpfelsäure | 1,8 |
| Calciumhypophosphit | 2 |
| Natrium-N, N-dimethylglycinat | 1,7 |

### Beispiel 4: Hautpflegecreme

| | |
|---|---|
| Caprylsäure-/Caprinsäuretriglycerid | 25 |
| Cocoglyceride | 25 |

| Wasser | ad 100 |
|---|---|
| Octyldodecylmyristat | 9 |
| Oleylerucat | 8 |
| Glycerylstearat | 5 |
| Glycerylbehenat | 5 |
| Jojoba-Ester | 5 |
| Sonnenblumenwachs | 1,5 |
| Hydriertes Rizinusöl | 3 |
| Benzylalkohol | 1 |
| Pentylenglykol | 0,5 |
| Butylenglykol | 0,5 |
| Mimosenwachs | 0,8 |
| Polyglycerin-3 | 0,25 |
| Bisabolol | 0,4 |
| Octenidindihydrochlorid | 0,05 |
| Hydroxyphenylpropamidobenzoesäure | 0,1 |
| Natrium-N,N-Dimethylglycinat | 1,5 |
| Äpfelsäure | 1,3 |
| Calciumhypophosphit | 1,7 |

### Beispiel 5: Hautpflegecreme

| Wasser | ad 100 |
|---|---|
| Glycerin | 5 |
| Cetylalkohol | 2 |
| Glycerylstearat | 1,5 |
| Squalan | 1,5 |
| Cetearylethylhexanoat | 1 |
| Octyldodecanol | 3 |
| Oleylerucat | 1,5 |
| Avocadoöl | 0,5 |
| Ethyllinoleat | 1,5 |
| PEG(40)stearat | 2 |
| Phenoxyethanol | 0,5 |
| Tocopherol | 0,5 |
| Calciumchlorid | 1,2 |
| Magnesiumchlorid | 3,7 |
| Phytosterole | 0,5 |
| Pflanzliches Öl | 0,5 |
| Sorbitantristearat | 0,5 |
| Olivenöl | 0,5 |
| Natrium-N,N-dimethylglycinat | 1 |
| Äpfelsäure | 0,5 |

Folgende Items fassen die Erfindung zusammen:
1. Zusammensetzung zur Behandlung des menschlichen Epithelgewebes umfassend
   (A) wenigstens eine organische Säure mit mindestens zwei funktionellen Gruppen, wobei wenigstens eine funktionelle Gruppe eine Carbonsäure ist, und
   (B) ein in Wasser lösliches anorganisches mehrwertiges Salz,
   wobei die Zusammensetzung einen pH-Wert zwischen 2,0 und 8,0 aufweist.
2. Zusammensetzung gemäß Anspruch 1, welche wenigstens eine Aminosäure, ein Aminosäurederivat (C) und/oder Mischungen davon umfasst.
3. Zusammensetzung gemäß einem der vorangegangenen Items, wobei das anorganische mehrwertige Salz mindestens ein Kation ausgewählt aus der Gruppe bestehend aus Mg2+, Ca2+, Sr2+, Zn2+, Al3+, Fe3+, La3+, oder beliebige Mischungen davon, umfasst.
4. Zusammensetzung gemäß einem der vorangegangenen Items, die wenigstens ein in Wasser lösliches Calcium- und/oder Magnesiumsalz, bevorzugt ein in Wasser lösliches Calciumsalz enthält.
5. Zusammensetzung gemäß einem der vorangegangenen Items, wobei es sich bei dem Salz (B) um das Calciumsalz der Phosphinsäure handelt, d.h. Calciumhypophosphit.
6. Zusammensetzung gemäß einem der vorangegangen Items, wobei die Zusammensetzung 0,001 bis 30,0 Gew.%, insbesondere 0,01 bis 10 Gew.%, bevorzugt 0,1 bis 2,0 Gew.%, besonders bevorzugt 0,3 bis 1,0 Gew.% Calciumhypophosphit enthält.
7. Zusammensetzung gemäß einem der vorangegangenen Items, wobei die funktionellen Gruppen ausgewählt sind aus -COOH, -(C=O)-, -C(=O)-O-, -OH, -SH, -NR₂, wobei R unabhängig ausgewählt ist aus H und Methyl, Ethyl oder Propyl, wobei H bevorzugt ist.
8. Zusammensetzung gemäß einem der vorangegangenen Items, wobei es sich bei der organischen Säure (A) um eine mehrwertige Carbonsäure mit pKs-Werten zwischen 1,5 und 10, bevorzugt zwischen 2,5 und 6,0, handelt.
9. Zusammensetzung gemäß einem der vorangegangenen Items, wobei der pH-Wert im Bereich zwischen 3 bis 7, bevorzugt im Bereich zwischen 4 bis 6, insbesondere im pH-Bereich zwischen 4,0 bis 5,0 liegt.
10. Zusammensetzung gemäß einem der vorangegangenen Items, wobei die organische Säure (A) ausgewählt ist aus der Gruppe bestehend aus Äpfelsäure, Maleinsäure, Weinsäure, Fumarsäure, Gallussäure, Gluconsäure, Glycolsäure, Mandelsäure, Milchsäure, Bernsteinsäure, Brenztraubensäure, L-(+)-Ascorbinsäure, D-Chinasäure, Citronensäure, Glutarsäure, Malonsäure und/oder Mischungen davon.
11. Zusammensetzung gemäß einem der vorangegangenen Items, wobei die Zusammensetzung 0,001 bis 10 Gew.%, insbesondere 0,01 bis 5% Gew.%, stärker bevorzugt 0,1 bis 2,0 Gew.-% an organischer Säure (A) enthält.
12. Zusammensetzung gemäß einem der vorangegangenen Items, wobei es sich bei der organischen Säure (A) um eine Dicarbonsäure handelt.
13. Zusammensetzung gemäß einem der vorangegangenen Items, welche das Aminosäurederivat (C) Dimethylglycin oder ein Dimethylglycinat enthält.
14. Zusammensetzung gemäß einem der vorangegangenen Items, welche 0,0001 bis 10 Gew.-%, bevorzugt 0,01 bis 5 Gew.-% Dimethylglycin oder eines Dimethylglycinates enthält.
15. Zusammensetzung gemäß einem der vorangegangenen Items, wobei die Zusammensetzung ferner Glycerin enthält.
16. Zusammensetzung gemäß Item 1, wobei die Zusammensetzung eine Viskosität zwischen 100 und 150.000 mP·sec aufweist.
17. Zusammensetzung gemäß einem der vorangegangenen Items, welche antibakterielle Substanzen enthält.
18. Zusammensetzung gemäß einem der vorangegangenen Items, wobei die Zusammensetzung zur topischen Anwendung ausgestaltet ist.
19. Zusammensetzung gemäß einem der vorangegangenen Items, welche ferner einen oder mehrere Wirkstoffe aus der Gruppe bestehend aus Biotin, Menthol, Taurin, Ectoin, Ubichinon-10, Echinacea, Panthenol, Nicotinsäureamid, Tocopherol, Tocopherolacetat, Harnstoff und Salz der Pyrrolidoncarbonsäure umfasst.
20. Zusammensetzung zur Behandlung des menschlichen Epithelgewebes umfassend
   (A) 0,01 bis 5 Gew.-% Äpfelsäure,
   (B) 0,01 bis 4 Gew.-% Calciumhypophosphit, und
   (C) 0,01 bis 5 Gew.-% Dimethylglycin und/oder ein Salz davon,
   wobei die Zusammensetzung einen pH-Wert zwischen 4,0 und 6,0 aufweist.
21. Zusammensetzung nach einem der Items 1 bis 20 zur Verwendung bei der Behandlung und/Vorbeugung von Schäden des menschlichen Epithelgewebes, bevorzugt eines Plattenepithelgewebes, insbesondere der Haut.
22 Zusammensetzung gemäß einem der vorangehenden Items zur Verwendung bei der Behandlung und/ oder Vorbeugung von Schuppenflechte (Psoriasis), Hautekzemen, atopische Dermatitis, Neurodermitis, Xerosis cutis (Hauttrockenheit), Pruritus (Jucken), Hautläsionen und/oder Atrophie.
23. Zusammensetzung gemäß einem der vorangehenden Items zur Verwendung bei der Behandlung und/oder Vorbeugung von Wunden und Narben und/oder Lichen Sclerosus.
24. Kosmetische Verwendung einer Zusammensetzung nach einem der Items 1-20.
25. Kosmetische Verwendung nach Item 24 bei aus rauer Haut, trockener Haut, sensibler Haut, Hautirritationen, Juckreiz und Pruritus.

## Patentansprüche

1. Zusammensetzung zur Behandlung des menschlichen Epithelgewebes umfassend
(A) wenigstens eine organische Säure mit mindestens zwei funktionellen Gruppen, wobei wenigstens eine funktionelle Gruppe eine Carbonsäure ist, und
(B) ein in Wasser lösliches anorganisches mehrwertiges Salz,
wobei die Zusammensetzung einen pH-Wert zwischen 2,0 und 8,0 aufweist.

2. Zusammensetzung gemäß Anspruch 1, welche wenigstens eine Aminosäure, ein Aminosäurederivat (C) und/oder Mischungen davon umfasst.

3. Zusammensetzung gemäß einem der vorangegangenen Ansprüche, wobei das anorganische mehrwertige Salz mindestens ein Kation ausgewählt aus der Gruppe bestehend aus Mg2+, Ca2+, Sr2+, Zn2+, Al3+, Fe3+, La3+, oder beliebige Mischungen davon, umfasst.

4. Zusammensetzung gemäß einem der vorangegangenen Ansprüche, die wenigstens ein in Wasser lösliches Calcium- und/oder Magnesiumsalz, bevorzugt ein in Wasser lösliches Calciumsalz enthält.

5. Zusammensetzung gemäß einem der vorangegangenen Ansprüche, wobei es sich bei dem Salz (B) um das Calciumsalz der Phosphinsäure handelt, d.h. Calciumhypophosphit, wobei die Zusammensetzung bevorzugt 0,001 bis 30,0 Gew.%, insbesondere 0,01 bis 10 Gew.%, mehr bevorzugt 0,1 bis 2,0 Gew.%, besonders bevorzugt 0,3 bis 1,0 Gew.% Calciumhypophosphit enthält.

6. Zusammensetzung gemäß einem der vorangegangenen Ansprüche, wobei es sich bei der organischen Säure (A) um eine mehrwertige Carbonsäure mit pKs-Werten zwischen 1,5 und 10, bevorzugt zwischen 2,5 und 6,0, handelt, bevorzugt um eine Dicarbonsäure, und/oder
wobei die organische Säure (A) ausgewählt ist aus der Gruppe bestehend aus Äpfelsäure, Maleinsäure, Weinsäure, Fumarsäure, Gallussäure, Gluconsäure, Glycolsäure, Mandelsäure, Milchsäure, Bernsteinsäure, Brenztraubensäure, L-(+)-Ascorbinsäure, D-Chinasäure, Citronensäure, Glutarsäure, Malonsäure und/oder Mischungen davon.

7. Zusammensetzung gemäß einem der vorangegangenen Ansprüche, wobei der pH-Wert im Bereich zwischen 3 bis 7, bevorzugt im Bereich zwischen 4 bis 6, insbesondere im pH-Bereich zwischen 4,0 bis 5,0 liegt.

8. Zusammensetzung gemäß einem der vorangegangenen Ansprüche, wobei die Zusammensetzung 0,001 bis 10 Gew.%, insbesondere 0,01 bis 5% Gew.%, stärker bevorzugt 0,1 bis 2,0 Gew.-% an organischer Säure (A) enthält.

9. Zusammensetzung gemäß einem der vorangegangenen Ansprüche, welche das Aminosäurederivat (C) Dimethylglycin oder ein Dimethylglycinat, bevorzugt
0,0001 bis 10 Gew.-%, besonders bevorzugt 0,01 bis 5 Gew.-% Dimethylglycin oder eines Dimethylglycinates enthält.

10. Zusammensetzung gemäß einem der vorangegangenen Ansprüche, wobei die Zusammensetzung zur topischen Anwendung ausgestaltet ist, wobei die Zusammensetzung bevorzugt eine Viskosität zwischen 100 und 150.000 mP·sec aufweist.

11. Zusammensetzung gemäß einem der vorangegangenen Ansprüche, welche ferner einen oder mehrere Wirkstoffe aus der Gruppe bestehend aus Biotin, Menthol, Taurin, Ectoin, Ubichinon-10, Echinacea, Panthenol, Nicotinsäureamid, Tocopherol, Tocopherolacetat, Harnstoff und Salz der Pyrrolidoncarbonsäure, und/oder
Glycerin, und/oder
antibakterielle Substanzen umfasst.

12. Zusammensetzung zur Behandlung des menschlichen Epithelgewebes umfassend
(A) 0,01 bis 5 Gew.-% Äpfelsäure,
(B) 0,01 bis 4 Gew.-% Calciumhypophosphit, und
(C) 0,01 bis 5 Gew.-% Dimethylglycin und/oder ein Salz davon,
wobei die Zusammensetzung einen pH-Wert zwischen 4,0 und 6,0 aufweist.

13. Zusammensetzung nach einem der Ansprüche 1 bis 12 zur Verwendung bei der Behandlung und/Vorbeugung von Schäden des menschlichen Epithelgewebes, bevorzugt eines Plattenepithelgewebes, insbesondere der Haut.

14. Zusammensetzung gemäß einem der vorangehenden Ansprüche zur Verwendung bei der Behandlung und/ oder Vorbeugung von Schuppenflechte (Psoriasis), Hautekzemen, atopische Dermatitis, Neurodermitis, Xerosis cutis (Hauttrockenheit), Pruritus (Jucken), Hautläsionen und/oder Atrophie, und/oder
zur Verwendung bei der Behandlung und/oder Vorbeugung von Wunden und Narben und/oder Lichen Sclerosus.

15. Kosmetische Verwendung einer Zusammensetzung nach einem der Ansprüche 1-12, bevorzugt bei aus rauer Haut, trockener Haut, sensibler Haut, Hautirritationen, Juckreiz und Pruritus.
